# EUROPEAN PATENT SPECIFICATION

(11) **EP 0 602 905 B1**
(45) Date of publication and mention of the grant of the patent: **05.06.2002**
(21) Application number: 93309952.5
(22) Date of filing: 10.12.1993
(51) Int. Cl.: A61K 7/02, A61K 7/027, A61K 7/48

(54) **Cosmetic compositions with improved transfer resistance**
Kosmetische Präparate mit verbessertem Übertragungswiderstand
Compositions cosmétiques ayant une résistance de transfert améliorée

(30) Priority: 15.12.1992 US 990716
(43) Date of publication of application: 22.06.1994
(73) Proprietor: Revlon Consumer Products Corporation, New York, NY 10022 (US)
(72) Inventor: Castrogiovanni, Anthony, Belford, New Jersey 07718 (US); Barone, Salvatore Joseph, Staten Island, New York 10306 (US); Krog, Ann, Red Bank, New Jersey 07701 (US); McCulley, Marion Larstanna, South River, New Jersey 08882 (US); Callelo, Joseph Frank, Union, New Jersey 07083 (US)
(74) Representative: W.P. THOMPSON & CO.

(56) References cited:
- EP-A- 0 362 860
- EP-A- 0 515 195
- EP-A- 0 590 192
- WO-A-93/17660
- US-A- 3 764 537
- US-A- 3 857 805
- US-A- 4 725 658
- US-A- 5 051 489
- US-A- 5 302 380
- STN INTERNATIONAL, KARLSRUHE. FILE CHEMICAL ABSTRACTS. AN=105:213946 & JP-A-61161211 (SHISEIDO CO. LTD.)
- STN INTERNATIONAL, KARLSRUHE. FILE CHEMICAL ABSTRACTS. AN=105:120498 & JP-A-61065809 (SHISEIDO CO. LTD.)
- STN INTERNATIONAL, KARLSRUHE. FILE CHEMICAL ABSTRACTS. AN=110:121023 & JP-A-62298512 (SHISEIDO CO. LTD.)
- STN INTERNATIONAL, KARLSRUHE. FILE CHEMICAL ABSTRACTS. AN=105:213941 & JP-A-61158913 (SHISEIDO CO. LTD.)
- STN INTERNATIONAL, KARLSRUHE. FILE CHEMICAL ABSTRACTS. AN=93:79880 & JP-A-55028906 (POLA CHEMICALS LTD)
- HAPPI HOUSEHOLD & PERSONAL PRODUCTS, vol.29, no.9, September 1992 page 44 and 113 V. VAN VALKENBURGH 'Novel silicone esters for cosmetics'

## Description

The invention relates to cosmetic make-up compositions for application to lips and skin.

Cosmetic compositions are generally defined as compositions suitable for application to the human body. Cosmetic compositions such as creams and lotions are used to moisturise skin and keep it in a smooth, supple condition. Pigmented cosmetic compositions such as make-up, for example, concealer, blusher, eye shadow, foundation, lipstick, etc. are used to colour or tint the skin and lips. Since adding colour is one of the most important reasons for wearing cosmetics, colour-containing cosmetics need to be very carefully formulated to provide maximum wear and effect.

JP61065809 seeks to provide a cosmetic which has excellent anti-running properties i.e. it has excellent water and oil resistance and good durability. The cosmetic comprises an organic silicone resin, a volatile oil and powder.

JP61158913 seeks to provide cosmetics which have good moisture resistance, perspiration resistance, oil resistance and lasting qualities. The cosmetics comprise an organic silicone resin, a volatile hydrocarbon oil and powder.

EP 0515195 A discloses liquid silicone esters. It exemplifies the use of these esters in compositions which contain substantial amounts of water and mentions that the esters exhibit improved water-resistance and greater substantivity.

Similarly HAPPI Household & Personal Products Industry, 29 (9): 44,113 (1992) discloses liquid silicone esters, in particular lauric and isostearic acid-based silicone esters. It states that these silicone esters have high substantivity.

US 5051489 A discloses silanol fatty ester compounds. These compounds are made by esterifying a fatty acid or carboxylic acid ester or anhydride with a silanol compound. They have lubricity and hydrophobicity.

JP 55028906 A relates to silicon waxes made from a siloxane and a fatty acid for use in lipsticks.

US 3764537 A relates to a synthetic petrolatum composition comprising polypropylene.

One of the long-standing problems with make-up, particularly lipstick, is the tendency of the cosmetic to blot or transfer from the skin onto other surfaces such as glassware, silverware, clothing, etc. This not only causes soiling of these surfaces, but forces the cosmetic wearer, especially lipstick user, to reapply cosmetic at fairly short intervals in order to maintain its effect, especially to keep the lips coloured.

Known cosmetics, particularly lipsticks, contain varying amounts of oil, wax and powder. However, all these hitherto-known compositions suffer from the disadvantages mentioned above.

It has now been found that incorporation of a volatile solvent and a silicone resin into such compositions mitigates the previously-mentioned disadvantages.

This invention therefore provides a cosmetic composition comprising oil, wax and powder, and optionally other excipients, characterised in that the composition comprises, by weight of the total composition,
(a) 1-70% volatile solvent having a viscosity substantially in the range of from 0.5 to 20 mPa.s at 25°C;
(b) 0.1-15% silicone ester wax which is lauryl trimethylolpropane siloxy silicate or isostearyl trimethylolpropane siloxy silicate, or a mixture thereof;
(c) 10-45% wax;
(d) 5-50% powder; and
(e) 1-30% oil.

The dependent claims specify preferred but optional features.

The composition of the invention provides a cosmetic composition which exhibits superior transfer resistance when compared with hitherto-known cosmetics.

In component (a), the term "volatile" means that the solvent has a measurable vapour pressure. The volatile solvents of the invention generally have a low viscosity, ranging from about 0.5 to 20 mPa.s at 25°C. Volatile solvents suitable for use in the compositions of the invention include volatile, low-viscosity silicone liquids such as cyclic silicones having the formula: 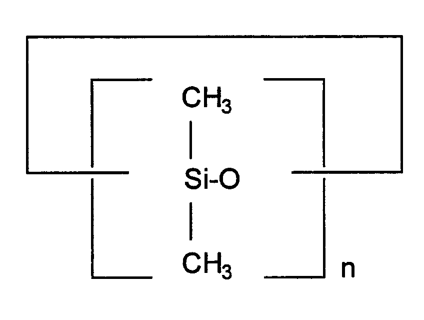 wherein n is an integer of from 3 to 7. Volatile linear polydimethylsiloxanes are also suitable and generally have from about 2 to 9 silicon atoms and are of the formula:

(CH₃)₃Si--O-[-Si(CH₃)₂--O-]ₙ--Si(CH₃)₃

wherein n is an integer of from 0 to 7. These silicones are available from various sources including Dow Corning Corporation, General Electric, etc. The Dow Corning cyclic silicones are sold under the tradenames Dow Corning 244, 245, 344, 345 and 200 fluids. These fluids comprise octamethylcyclotetrasiloxane, decamethylcyclopentasiloxane, hexamethyldisiloxane, or mixtures thereof. Also suitable as the volatile solvent are various C₈-C₂₀ isoparaffins such as C₁₂ isoparaffin made by The Permethyl Corporation having the registered trade mark Permethyl 99A (isododecane), distributed for The Permethyl Corporation by Presperse Inc., South Plainfield, New Jersey, U.S.A. Various C₁₆ isoparaffins are commercially available, such as isohexadecane (having the registered trade mark Permethyl 101A) and are also suitable. It is generally preferred that the volatile solvent component be a mixture of volatile silicones and C₈₋₂₀ isoparaffins. A ratio of from 10:1 to 1:10, respectively, is suitable. Preferably, the formulation comprises 35-60% of the volatile solvent. Therefore, a preferred formulation of the invention comprises 35-60% of a volatile solvent itself comprising a 10:1 to 1:10 ratio of cyclic silicones and C₈₋₂₀ isoparaffins.

For background information about silicone ester waxes reference may be made to US patent specification no. 4 725 658 which is incorporated herein by reference. The silicone ester waxes generally have a melting point of from about 40 to 90°C.

The waxes or wax-like materials (also known in the art as "waxes") of the invention generally have a melting point in the range of from 35-120°C. Waxes in this category include synthetic wax, ceresin, paraffin, ozokerite, illipe butter, beeswax, carnauba, microcrystalline, lanolin, lanolin derivatives, candelilla, cocoa butter, shellac wax, spermaceti, stearyl alcohol, bran wax, capok wax, sugar cane wax, montan wax, whale wax, bayberry wax, or mixtures thereof. Preferably, the formulation of the invention comprises about 10-30% wax, more preferably as a mixture of waxes.

The powder component of the invention is generally a dry, particulate matter having a particle size of 0.02-50 micrometres. The particulate matter may be coloured or non-coloured (for example, white) and, in particular, pigments are considered as "powder" for the purposes of this invention.

Suitable non-pigmented powders include bismuth oxychloride, titanated mica, fumed silica, spherical silica beads, polymethylmethacrylate beads, micronized teflon, boron nitride, acrylate polymers, aluminum silicate, aluminum starch octenylsuccinate, bentonite, calcium silicate, cellulose, chalk, corn starch, distomaceous earth, fuller's earth, glyceryl starch, hectorite, hydrated silica, kaolin, magnesium aluminum silicate, magnesium carbonate, magnesium hydroxide, magnesium oxide, magnesium silicate, magnesium trisilicate, maltodextrin, montmorillonite, microcrystalline cellulose, rice starch, silica, talc, mica, titanium dioxide, zinc laurate, zinc myristate, zinc neodecanoate, zinc rosinate, zinc stearate, polyethylene, alumina, attapulgite, calcium carbonate, calcium silicate, dextran, kaolin, nylon, silica silylate, silk powder, serecite, soy flour, tin oxide, titanium hydroxide, trimagnesium phosphate, walnut shell powder, or mixtures thereof. The above-mentioned powders may be surface-treated with lecithin, amino acids, mineral oil, silicone oil or various other agents, either alone or in combination, which coat the powder surface and render the particles hydrophobic in nature.

The powder component may also comprise various organic and inorganic pigments. The organic pigments are generally various aromatic types including azo, indigoid, triphenylmethane, anthraquinone, and zanthine dyes which are designated as D&C and FD&C blues, browns, greens, oranges, reds, yellows, etc. Inorganic pigments generally consist of insoluble metallic salts of certified colour additives, referred to as the Lakes or iron oxides.

The percentage of pigments used in the powder component will depend upon the type of cosmetic being formulated. Blushers, eyeshadows, lipsticks and similar cosmetics will contain higher percentages of pigment in the powder phase, usually ranging from 5-50% of the total cosmetic composition. Generally, the powder:pigment ratio ranges from 1:20 to 20:1.

Preferably, the invention comprises 10-30% of a powder component, especially wherein the non-pigment powder component comprises about 10-20% of the total composition and the pigment component comprises 1-10% of the total composition.

The composition of the invention also contains an oil, preferably comprising a mixture of low viscosity and high viscosity oils.

Suitable low viscosity oils have a viscosity of 5 to 100 mPa.s at 25°C, and are generally esters having the structure RCO-OR' wherein RCO represents a carboxylic acid radical and wherein OR' is an alcohol residue. Examples of these low viscosity oils include isotridecyl isononanoate, PEG-4 diheptanoate, where PEG-4 is a polymer of ethylene oxide of formula H(OCH₂CH₂)ₙOH wherein n has an average value of 4, isostearyl neopentanoate, tridecyl neopentanoate, cetyl octanoate, cetyl palmitate, cetyl ricinoleate, cetyl stearate, cetyl myristate, coco-dicaprylate/caprate, decyl isostearate, isodecyl oleate, isodecyl neopentanoate, isohexyl neopentanoate, octyl palmitate, dioctyl malate, tridecyl octanoate, myristyl myristate, dioctyl malate ester, octyldodecanol, or mixtures of octyldodecanol, acetylated lanolin alcohol, cetyl acetate, isododecanol, polyglyceryl-3-diisostearate, or mixtures of any of these.

Suitable high viscosity surface oils generally have a viscosity of 200-1,000,000 mPa.s at 25°C, preferably a viscosity of 100,000-250,000 mPa.s at 25°C. Surface oils include castor oil, lanolin and lanolin derivatives, triisocetyl citrate, sorbitan sesquioleate, C₁₀₋₁₈ triglycerides, caprylic/capric/triglycerides, coconut oil, corn oil, cottonseed oil, glyceryl triacetyl hydroxystearate, glyceryl triacetyl ricinoleate, glyceryl trioctanoate, hydrogenated castor oil, linseed oil, mink oil, olive oil, palm oil, illipe butter, rapeseed oil, soybean oil, sunflower seed oil, tallow, tricaprin, trihydroxystearin, triisostearin, trilaurin, trilinolein, trimyristin, triolein, tripalmitin, tristearin, walnut oil, wheat germ oil, cholesterol, or mixtures thereof.

The ratio of low viscosity to high viscosity oils in the oil phase is preferably in the range of from 1:15 to 15:1, more preferably 1:10 to 10:1, respectively. The preferred formulation of the invention comprises 1-20% of a mixture of low viscosity and high viscosity surface oils.

Especially preferred compositions are those comprising:
a) 35-60% of the volatile solvent;
b) 3-10% of the silicone ester wax which is lauryl trimethylolpropane siloxy silicate or isostearyl trimethylolpropane siloxy silicate, or a mixture thereof;
c) 10-30% of the wax;
d) 10-30% of the powder component comprised, by weight of the total composition, of 10-20% non-pigment powder and 1-10% pigment powder; and
e) 5-20% of a mixture of low viscosity and high viscosity surface oils.

Other ingredients may also be added to the above composition including various types of amorphous or atactic polypropylene, and preservatives or antioxidants, and the like.

Preferably, amorphous or atactic polypropylene is incorporated in the range of from about 0.1-10%. Suitable polypropylenes are atactic polypropylenes having about 50-100% atactic content, 0.1-15% crystallinity, and a molecular weight of about 1,000-10,000 wherein the term "molecular weight" means average number molecular weight; the term "atactic content" means that the polypropylene polymeric structure is random or without orientation, as opposed to isotactic or syndiotactic polymers which exhibit specific orientation and structural regularity. Isotactic and syndiotactic polymers, due to their structural regularity, can easily cross-link to form a crystalline network whereas the structural irregularity of atactic polymers precludes appreciable polymeric cross linking: 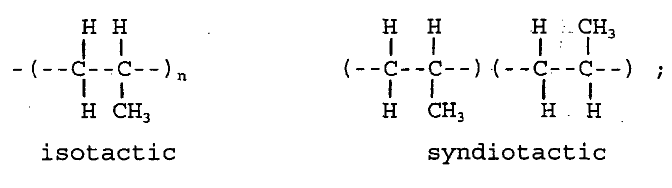 and
the term "crystallinity" refers to the degree of cross-linking of the polypropylene polymer. Crystallinity is directly proportional to the degree of polymeric cross-linking, and polymers which exhibit extensive cross-linking are highly crystalline in nature.

Preferably, the atactic polypropylene is selected from the group consisting of an atactic polypropylene having a softening point of 20°C and a molecular weight of 2,000; an atactic polypropylene having a softening point of 135°C and a molecular weight of 5,600; and an atactic polypropylene having a softening point of 150°C and a molecular weight of about 4,400.

Suitable preservatives include those such as the parabens, butylated hydroxy toluene (BHT), butylated hydroxy anisole (BHA), and the like. Generally, 0.01-5% preservative is suitable.

The present invention further provides a cosmetic such as a concealer, blusher, eye shadow, foundation or lipstick, which cosmetic comprises a composition as hereinbefore described in association with a suitable receptacle therefor.

The present invention further provides a method for making-up skin or lips, which method comprises topical administration of a composition or cosmetic as hereinbefore described.

The present invention further provides a process for preparing a cosmetic composition as hereinbefore described, which process comprises bringing into intimate physical admixture
(a) 1-70% of a volatile solvent having a viscosity substantially in the range of from 0.5 to 20 mPa.s at 25°C;
(b) 0.1-15% silicone resin which is lauryl trimethylolpropane siloxy silicate or isostearyl trimethylolpropane siloxy silicate, or a mixture thereof;
(c) 10-45% of a wax having a melting point in the range of from 35 to 120°C;
(d) 5-50% powder; and
(e) 1-30% oil
The cosmetic compositions according to the invention provide a novel finish which exhibits superior transfer resistance when compared with hitherto-known cosmetic compositions. The term "superior transfer resistance" means that when the cosmetic composition is applied to skin or lips it exhibits from 10-100%, preferably 30-100%, improvement in transfer resistance when compared with a hitherto-known formulation. When the cosmetic is a lipstick, transfer resistance can be measured by the "Kiss Test" as described in Example 4.

The invention will be further illustrated by the following examples.

### EXAMPLE 1

A transfer resistant lipstick composition with shine was made according to the following formula:

| | w.w% | |
|---|---|---|
| | (a) | (b) |
| Synthetic wax | 6.00 | 6.60 |
| Ceresin | 4.00 | 4.00 |
| Paraffin | 3.00 | 3.00 |
| Isododecane | 10.00 | 10.00 |
| Cetyl acetate:acetylated lanolin alcohol (90:10) | 5.00 | 5.00 |
| Methyl paraben | 0.30 | 0.30 |
| Propyl paraben | 0.10 | 0.10 |
| BHA | 0.10 | 0.10 |
| D&C Red Calcium Lake | 4.00 | 3.00 |
| FD&C Yellow 5 Aluminum Lake | 3.00 | ---- |
| Titanium dioxide/mica | 5.00 | ---- |
| Titanium dioxide/mica/iron oxide | 3.00 | ---- |
| Bismuth oxychloride | 10.00 | 15.00 |
| Cyclomethicone | 41.50 | 40.40 |
| Isostearyl trimethylolpropane siloxy silicate | 5.00 | 5.00 |

### EXAMPLE 2

A transfer resistant lipstick composition was made according to the following formula:

| | w.w% |
|---|---|
| Cococaprylate/caprate | 2.70 |
| Cetyl acetate:acetylated lanolin | |
| alcohol | 1.00 |
| Isostearyltrimethylol propane | |
| siloxysilicate | 7.00 |
| Methylparaben | 0.30 |
| Propylparaben | 0.10 |
| BHA | 0.10 |
| Synthetic wax | 6.60 |
| Ceresin wax | 4.00 |
| Paraffin wax | 3.00 |
| Ozokerite wax | 1.00 |
| Illipe butter | 0.20 |
| Octyldodecanol, trilaurin phospholipids, | |
| cholesterol, glycosphingolipids | 0.50 |
| Bismuth oxychloride | 9.00 |
| Dimethicone treated mica | 2.50 |
| Mica/titanium dioxide | 2.50 |
| Cyclomethicone | 41.40 |
| Isododecanol | 9.00 |
| Polypropylene (atactic) | 0.10 |
| Lanolin oil | 3.50 |
| Trioctyldodecyl citrate | 2.00 |
| pigment solids* | 3.50 |
| * D&C red 7 Calcium Lake | 0.90 |
| D&C red 7 Barium Lake | 2.30 |
| FD&C yellow 6 Aluminum Lake | 0.30 |

The lipstick formulations of Examples 1 and 2 were made by grinding a portion of the powder (non-pigmented and pigmented) ingredients with some of the volatile solvent and silicone ester wax. Next, the waxes and oils were added with heating. The remainder of the powder component was then added. The mixture was then stirred before pouring into moulds and left to cool.

### EXAMPLE 3

A transfer resistant eyeshadow, blusher and concealer were made according to the following compositions:

| | w/w% | | |
|---|---|---|---|
| | eyeshadow | blusher | concealer |
| Cococaprylate/caprate | 2.700 | 2.700 | 2.700 |
| Cetyl acetate/acetylated lanolin alcohol | 1.000 | 1.000 | 1.000 |
| Isostearyl trimethylol propanesiloxysilicate | 7.000 | 7.000 | 7.000 |
| Synthetic wax | 6.600 | 6.600 | 6.600 |
| Ceresin wax | 4.000 | 4.000 | 4.000 |
| Paraffin wax | 3.000 | 3.000 | 3.000 |
| Ozokerite | 1.000 | 1.000 | 1.000 |
| Octyldodecanol/trilaurin phospholipid/cholesterol/glycosphingolipids | 0.500 | 0.500 | 0.500 |
| Illipe butter | 0.200 | 0.200 | 0.200 |
| Polypropylene (atactic) | 0.100 | 0.100 | 0.100 |
| Methyl paraben | 0.300 | 0.300 | 0.300 |
| Propyl paraben | 0.100 | 0.100 | 0.100 |
| BHA | 0.100 | 0.100 | 0.100 |
| Lanolin oil | 3.500 | 3.500 | 3.500 |
| D&C red 7 calcium lake | ----- | 2.100 | ----- |
| FD&C yellow 5 aluminum lake | ----- | 0.800 | ----- |
| Red iron oxide | 2.100 | ----- | 2.100 |
| Yellow iron oxide | 0.800 | ----- | 0.800 |
| Black iron oxide | 0.500 | 0.500 | 0.100 |
| Titanium dioxide | 0.100 | 0.100 | 0.100 |
| Bismuth oxychloride | 3.000 | 3.000 | 3.000 |
| Titanium dioxide/mica | 9.000 | 9.000 | 9.000 |
| Cyclomethicone | 41.400 | 41.400 | 41.400 |
| Mico/dimethicone | 2.000 | 2.000 | 2.000 |
| Isododecane | 9.000 | 9.000 | 9.000 |
| Trioctyldodecyl citrate | 2.000 | 2.000 | 2.000 |

The above cosmetic compositions were made by first mixing all the dry ingredients. The waxes and oils were added with heating. The volatile solvent and silicone ester wax were next added, followed by the remaining ingredients. The mixture was stirred before being poured into the appropriate moulds and allowed to cool.

### EXAMPLE 4

Two groups of thirty nine panelists were asked to compare a lipstick formulation of the invention according to Example 2 hereof ("A") with a known, commercially-available lipstick formulation ("B"), having a formulation as specified at the end of this example.

### Formulations A and B

| A | | B |
|---|---|---|
| 1-70% volatile solvent | | -* |
| 0.1-1.5% silicone resin | | - |
| 10-45% wax | | (candelilla wax |
| | 10-45% | (carnauba wax |
| | | (ceresin |
| | | (paraffin |
| 5-50% powder | 5-50% | (mica |
| | | (titanium dioxide |
| | | (reds and yellow |
| | | |
| 1-30% oil | 1-30%* | (octyl palmitate |
| | | (lanolin oil |
| | | (castor oil |
| | | (cetyl acetate |

| | | |
|---|---|---|
| (*: With the balance of the formulation made up by a higher % of natural oils, in the absence of volatile solvent). | | |

The first group of thirty nine panelists was asked to use lipstick "A" for one week in place of their current lipstick brand. The second group of thirty nine panelists was asked to use formulation "B" for one week in place of their current lipstick brand. The panelists were then asked a series of questions as follows:

| Did the lipstick product bleed? | | |
|---|---|---|
| | A | B |
| Yes | 1 | 8 |
| No | 38 | 31 |

| If so, after how long? | | |
|---|---|---|
| | A | B |
| ½ hour | - | 1 |
| 1 hour | - | 1 |
| 2 hours | - | 4 |
| Other (3-5 hours) | 1 | 2 |

| Did the lipstick leave a rim on coffee cups or cigarettes? | | |
|---|---|---|
| | A | B |
| Yes | 15 | 36 |
| No | 24 | 3 |

| Would you consider this test lipstick to be "long wearing"? | | |
|---|---|---|
| | A | B |
| Yes | 34 | 36 |
| No | 5 | 21 |

### KISS TEST

Panelists were asked to apply the lipstick, wait 5 minutes, then kiss their hand. The panelists were then asked if the lipstick "left hardly a trace" of colour on their hand. They reported as follows:

| | A | B |
|---|---|---|
| Yes | 34 | 6 |
| No | 5 | 33 |

The above results show that the lipstick formulation of the invention exhibited superior transfer resistance when compared to a commercial lipstick formulation. Approximately 87% of the panelists agreed that lipstick "A" left hardly a trace of colour on their hand whereas only 15% of panelists agreed that sample "B" (a traditional lipstick) left hardly a trace of colour on their hand. The degree of improvement seen with sample A is approximately 72%.

| Further details of Formulation B | |
|---|---|
| | w/w% |
| Candelilla wax | 1-5 |
| Carnauba | 1-5 |
| Ceresin | 1-5 |
| Paraffin | 1-5 |
| Propylene glycol ricinoleate | 1-10 |
| Octyl hydroxystearate | 5-15 |
| PVP hexadecene copolymer | 1-5 |
| UV absorbers | 0.5-5 |
| Cetyl acetate:acetylated lanolin alcohol (90:10) | 10-15 |
| Octyl palmitate | 5-10 |
| Lanolin oil | 5-25 |
| Preservatives | 0.1-3 |
| Castor oil | 5-25 |
| Acrylates copolymer | 0.01-5 |
| Pigments | 3-20 |
| Fragrance | 0.1-1 |

## Claims

1. A cosmetic composition comprising oil, wax and powder, and optionally other excipients, **characterised in that** the composition comprises, by weight of the total composition:
(a) 1-70% volatile solvent having a viscosity substantially in the range of from 0.5 to 20mPa.s at 25°C;
(b) 0.1-15% of a silicone ester wax which is lauryl trimethylolpropane siloxy silicate or isostearyl trimethylolpropane siloxy silicate, or a mixture thereof;
(c) 10-45% wax;
(d) 5-50% powder; and
(e) 1-30% oil.

2. A composition according to Claim 1 wherein the volatile solvent is a cyclic silicone having the formula: 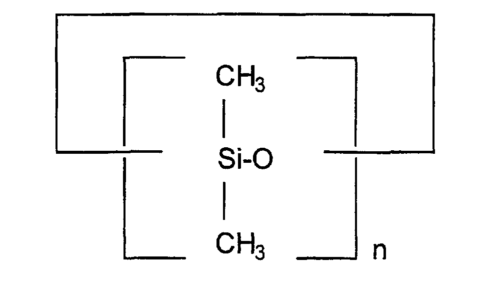 wherein n is an integer of from 3-7; or a volatile linear polydimethylsiloxane having 2 to 9 silicon atoms and of the general formula:
(CH₃)₃Si--O-[Si(CH₃)₂--O-]ₙ---Si(CH₃)₃
wherein n is an integer of from 0-7; or a C₈-C₂₀ isoparaffin; or mixtures thereof.

3. A composition according to Claim 2 wherein the cyclic silicone is cyclomethicone.

4. A composition according to Claim 2 or 3 wherein the volatile solvent is itself comprised of a 10:1 to 1:10 ratio of cyclic silicones and C₈₋₂₀ isoparaffins.

5. A composition according to any one of Claims 1 to 4 wherein the wax of component (c) has a melting point in the range of from 35-120°C.

6. A composition according to any one of Claims 1 to 5 wherein the wax of component (c) is synthetic wax, ceresin, paraffin, ozokerite, illipe butter, beeswax, carnauba, microcrystalline, lanolin, lanolin derivatives, candelilla, cocoa butter, shellac wax, spermaceti, stearyl alcohol, bran wax, capok wax, sugar cane wax, montan wax, whale wax or bayberry wax, or a mixture thereof.

7. A composition according to any one of Claims 1 to 6 wherein the powder comprises a dry particulate matter having a particle size in the range of from 0.02-50 micrometers.

8. A composition according to any one of Claims 1 to 7 wherein the ratio of non-pigment powder to pigment in the powder is in the range of from 1:20 to 20:1, respectively.

9. A composition according to any one of Claims 1 to 8 wherein the oil comprises a low viscosity oil, a high viscosity oil or a mixture thereof wherein the low viscosity oil has a viscosity in the range of from 5 to 100 mPa.s at 25°C and wherein the high viscosity oil has a viscosity in the range of from 200-1,000,000 mPa.s at 25°C.

10. A composition according to any one of Claims 1 to 9 wherein the oil comprises a mixture of low viscosity and high viscosity oils in the ratio ranging from 1:15 to 15:1, respectively.

11. A composition according to any one of Claims 1 to 10 wherein the oil incorporates a low viscosity surface oil selected from isotridecyl isononanoate, PEG-4 diheptanoate, isostearyl neopentanoate, tridecyl neopentanoate, cetyl octanoate, cetyl palmitate, cetyl ricinoleate, cetyl stearate, cetyl myristate, cocodicaprylate/caprate, decyl isostearate, isodecyl oleate, isodecyl neopentanoate, isohexyl neopentanoate, octyl palmitate, dioctyl malate, tridecyl octanoate, myristyl myristate, dioctyl malate ester, octyldodecanol, or mixtures of octyldodecanol, acetylated lanolin alcohol, cetyl acetate, isododecanol and polyglyceryl-3-diisostearate, and mixtures of any of these.

12. A composition according to any one of Claims 1 to 11 which further comprises an amorphous or atactic polypropylene having 50-100% atactic content, 0.1-15% crystallinity, and a molecular weight of 1,000-10,000.

13. A composition according to Claim 12, wherein the atactic polypropylene is selected from the group consisting of an atactic polypropylene having a softening point of 20°C and a molecular weight of 2,000; an atactic polypropylene having a softening point of 135°C and a molecular weight of 5,600; and an atactic polypropylene having a softening point of 150°C and a molecular weight of 4,400.

14. A composition according to any one of Claims 1 to 13 comprising:
(a) 35-60% volatile solvent which is itself comprised of a 10:1 to 1:10 ratio of cyclic silicones and C₈₋₂₀ isoparaffins;
b) 3-10% of lauryl trimethylolpropane siloxy silicate or isostearyl trimethylolpropane siloxy silicate, or a mixture thereof;
c) 10-30% wax;
d) 10-30% powder component comprising, by weight of the total composition, of 10-20% non-pigment powder and 1-10% pigment powder; and
e) 5-20% mixture of low viscosity and high viscosity surface oils.

15. A cosmetic such as a concealer, blusher, eye shadow, foundation or lipstick, which cosmetic comprises a composition according to any one of Claims 1 to 14 in association with a suitable receptacle therefor.

16. A method for making-up skin or lips, which method comprises topical administration of a composition or cosmetic according to any one of Claims 1 to 14.

17. A process for preparing a cosmetic composition according to any one of Claims 1 to 14, which process comprises bringing into intimate physical admixture:
(a) 1-70% volatile solvent having a viscosity substantially in the range of from 0.5 to 20 mPa.s at 25°;
(b) 0.1-15% silicone ester wax which is lauryl trimethylolpropane siloxy silicate or isostearyl trimethylolpropane siloxy silicate, or a mixture thereof;
(c) 10-45% wax having a melting point in the range of from 35 to 120°C;
(d) 5-50% powder; and
(e) 1-30% oil
and, if desired, allowing the mixture so produced to set in a mould.

## Patentansprüche

1. Kosmetikzusammensetzung, die Öl, Wachs und Puder und gegebenenfalls andere Träger umfasst, **dadurch gekennzeichnet, dass** die Zusammensetzung bezogen auf das Gewicht der Gesamtzusammensetzung umfasst:
(a) 1 bis 70 % flüchtiges Lösungsmittel mit einer Viskosität im Wesentlichen im Bereich von 0,5 bis 20 mPa·s bei 25°C,
(b) 0,1 bis 15 % Silikonesterwachs, das Lauryltrimethylolpropansiloxysilikat oder Isostearyltrimethylolpropan siloxysilikat oder eine Mischung derselben ist,
(c) 10 bis 45 % Wachs,
(d) 5 bis 50 % Puder und
(e) 1 bis 30 % Öl.

2. Zusammensetzung nach Anspruch 1, bei der das flüchtige Lösungsmittel cyclisches Silikon mit der Formel: 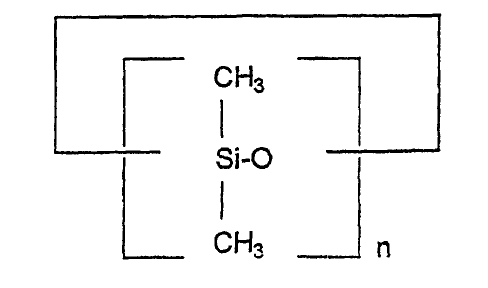 ist, in der n eine Zahl von 3 bis 7 ist, oder flüchtiges lineares Polydimethylsiloxan mit 2 bis 9 Siliciumatomen und der allgemeinen Formel:
(CH₃)₃Si-O-[Si(CH₃)₂-O]ₙ-Si (CH)₃
ist, in der n eine Zahl von 0 bis 7 ist, oder ein C₈- bis C₂₀-Isoparaffin oder Mischungen derselben ist.

3. Zusammensetzung nach Anspruch 2, bei der das cyclische Silikon Cyclomethicon ist.

4. Zusammensetzung nach Anspruch 2 oder 3, bei der das flüchtige Lösungsmittel selbst aus cyclischen Silikonen und C₈ - bis C₂₀-Isoparaffinen in einem Verhältnis von 10:1 bis 1:10 zusammengesetzt ist.

5. Zusammensetzung nach einem der Ansprüche 1 bis 4, bei der das Wachs von Komponente (c) einen Schmelzpunkt im Bereich von 35 bis 120°C hat.

6. Zusammensetzung nach einem der Ansprüche 1 bis 5, bei der das Wachs von Komponente (c) synthetisches Wachs, Ceresin, Paraffin, Ozokerit, Illipebutter, Bienenwachs, Carnauba, mikrokristallines Wachs, Lanolin, Lanolinderivate, Candelilla, Kakaobutter, Shellackwachs, Spermazet (Walrat), Stearylalkohol, Kleiewachs, Capokwachs, Zuckerrohrwachs, Montanwachs, Walwachs oder Myrtenwachs oder eine Mischung derselben ist.

7. Zusammensetzung nach einem der Ansprüche 1 bis 6, bei der das Puder trockenes teilchenförmiges Material mit einer Partikelgröße im Bereich von 0,02 bis 50 µm umfasst.

8. Zusammensetzung nach einem der Ansprüche 1 bis 7, bei der das Verhältnis von Nicht-Pigmentpuder zu Pigment in dem Puder im Bereich von jeweils 1:20 bis 20:1 liegt.

9. Zusammensetzung nach einem der Ansprüche 1 bis 8, bei der das Öl Öl mit niedriger Viskosität, Öl mit hoher Viskosität oder eine Mischung derselben umfasst, wobei das Öl mit niedriger Viskosität eine Viskosität im Bereich von 5 bis 100 mPa·s bei 25°C hat und das Öl mit hoher Viskosität eine Viskosität im Bereich von 200 bis 1 000 000 mPa·s bei 25°C hat.

10. Zusammensetzung nach einem der Ansprüche 1 bis 9, bei der das Öl eine Mischung aus Ölen mit niedriger und mit hoher Viskosität im Verhältnis im Bereich von jeweils 1:15 bis 15:1 umfasst.

11. Zusammensetzung nach einem der Ansprüche 1 bis 10, bei der das Öl Oberflächenöl mit niedriger Viskosität ausgewählt aus Isotridecylisononanoat, PEG-4-diheptanoat, Isostearylneopentanoat, Tridecylneopentanoat, Cetyloctanoat, Cetylpalmitat, Cetylricinoleat, Cetylstearat, Cetylmyristat, Cocodicaprylat/-caprat, Decylisostearat, Isodecyloleat, Isodecylneopentanoat, Isohexylneopentanoat, Octylpalmitat, Dioctylmalat, Tridecyloctanoat, Myristylmyristat, Dioctylmalatester, Octyldodecanol oder Mischungen von Octyldodecanol, acyliertem Lanolinalkohol, Cetylacetat, Isododecanol und Polyglyceryl-3-diisostearat und Mischungen von beliebigen von diesen umfasst.

12. Zusammensetzung nach einem der Ansprüche 1 bis 11, die ferner amorphes oder ataktisches Polypropylen mit 50 bis 100 % ataktischem Gehalt, 0,1 bis 15 % Kristallinität und einem Molekulargewicht von 1 000 bis 10 000 umfasst.

13. Zusammensetzung nach Anspruch 12, bei der das ataktische Polypropylen ausgewählt ist aus der Gruppe bestehend aus ataktischem Polypropylen mit einem Erweichungspunkt von 20°C und einem Molekulargewicht von 2 000, ataktischem Polypropylen mit einem Erweichungspunkt von 135°C und einem Molekulargewicht von 5 600 und ataktischem Polypropylen mit einem Erweichungspunkt von 150°C und einem Molekulargewicht von 4 400.

14. Zusammensetzung nach einem der Ansprüche 1 bis 13, umfassend:
(a) 35 bis 60 % flüchtiges Lösungsmittel, das selbst aus cyclischen Silikonen und C₈- bis C₂₀-Isoparaffinen in einem Verhältnis von 10:1 bis 1:10 zusammengesetzt ist,
b) 3 bis 10 % Lauryltrimethylolpropansiloxysilikat oder Isostearyltrimethylolpropansiloxysilikat oder eine Mischung derselben,
c) 10 bis 30 % Wachs,
d) 10 bis 30 % Puderkomponente, die bezogen auf das Gewicht der Gesamtzusammensetzung 10 bis 20 % Nicht-Pigmentpuder und 1 bis 10 % Pigmentpuder umfasst, und
e) 5 bis 20 % Mischung von Oberflächenölen mit niedriger und hoher Viskosität.

15. Kosmetikum wie Abdeckmittel, Rouge, Lidschatten, Grundierung oder Lippenstift, wobei das Kosmetikum eine Zusammensetzung gemäß einem der Ansprüche 1 bis 14 zusammen mit einem geeigneten Aufnahmegefäß dafür umfasst.

16. Verfahren zum Schminken von Haut oder Lippen, das topische Verabreichung einer Zusammensetzung oder eines Kosmetikums nach einem der Ansprüche 1 bis 14 umfasst.

17. Verfahren zur Herstellung einer Kosmetikzusammensetzung nach einem der Ansprüche 1 bis 14, bei dem in innige physikalische Mischung gebracht werden:
(a) 1 bis 70 % flüchtiges Lösungsmittel mit einer Viskosität im Wesentlichen im Bereich von 0,5 bis 20 mPa·s bei 25°C,
(b) 0,1 bis 15 % Silikonesterwachs, das Lauryltrimethylolpropansiloxysilikat oder Isostearyltrimethylolpropansiloxysilikat oder eine Mischung derselben ist,
(c) 10 bis 45 % Wachs mit einem Schmelzpunkt im Bereich von 35 bis 120°C,
(d) 5 bis 50 % Puder, und
(e) 1 bis 30 % Öl,
und gewünschtenfalls die so hergestellte Mischung in einer Form fest werden gelassen wird.

## Revendications

1. Un composition cosmétique comprenant de l'huile, une cire et de la poudre, et le cas échéant d'autres excipients, **caractérisée en ce que** la composition comprend, en poids par rapport à la composition totale:
(a) 1 à 70 % de solvant volatile ayant une viscosité sensiblement dans la fourchette de 0,5 à 20 mPa.s, à 25°C;
(b) 0,1 à 15% d'une cire d'ester de silicone qui est le lauryltriméthylolpropanesiloxy silicate ou l'isostéaryltriméthylolpropane siloxy silicate ou leur mélange ;
(c) 10 à 45 %de cire ;
(d) 5 à 50 % de poudre ; et
(e) 1 à 30 % d'huile.

2. Une composition selon la revendication 1, dans laquelle le solvant volatile est une silicone cyclique ayant la formule : 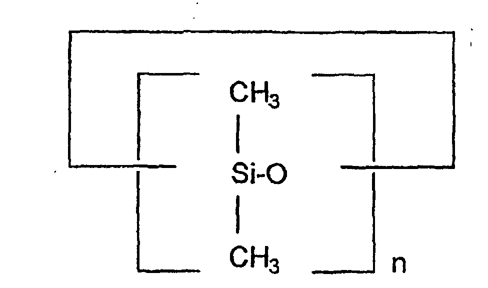 dans laquelle n est un entier de 3 à 7 ; ou un polydiméthylsiloxane linéaire volatil comportant 2 à 9 atomes de silicium et de formule générale :
(CH₃)₃Si--O-[Si(CH₃)₂--O-]ₙ---Si (CH₃)₃
dans laquelle n est un entier de 0 à 7; ou bien une isoparaffine en C₈ à C₂₀; ou leurs mélanges.

3. Une composition selon la revendication 2, dans laquelle la silicone cyclique est la cyclométhicone.

4. Une composition selon la revendication 2 ou 3, dans laquelle le solvant volatil lui-même comprend un rapport 10/1 à 1/10 de silicones cycliques et d'isoparaffines en C₈ à C₂₀.

5. Une composition selon l'une quelconque des revendications 1 à 4, dans laquelle la cire du composant (c) a un point de fusion dans la fourchette de 35 à 120°C.

6. Une composition selon l'une quelconque des revendications 1 à 5, dans laquelle la cire du composant (c) est une cire synthétique, une cérésine, une paraffine, une ozokérite, un beurre d'illipe, de la cire d'abeille, du carnauba, de la lanoline microcristalline, des dérivés de lanoline, du candelilla, du beurre de cacao, de la cire de shellac, du spermacéne, de l'alcool stéarylique, de la cire de son, de la cire de capok, de la cire de canne à sucre, de la cire minérale, de la cire de baleine ou de la cire de Pimenta racemosa, ou leurs mélanges.

7. Une composition selon l'une quelconque des revendications 1 à 6, dans laquelle la poudre comprend une matière particulaire sèche ayant une taille particulaire dans la fourchette de 0,02 à 50 micromètres.

8. Une composition selon l'une quelconque des revendications 1 à 7, dans laquelle le rapport de la poudre non pigmentée au pigment dans la poudre est dans la fourchette de 1/20 à 20/1, respectivement.

9. Une composition selon l'une quelconque des revendications 1 à 8, dans laquelle l'huile comprend une huile de basse viscosité, une huile de haute viscosité ou un de leurs mélanges, dans lequel l'huile de basse viscosité a une viscosité dans la fourchette de 5 à 100 mPa.s à 25°C et dans laquelle l'huile de haute viscosité a une viscosité dans la fourchette de 200 à 1 000 000 mPa.s à 25°C.

10. Une composition selon l'une quelconque des revendications 1 à 9, dans laquelle l'huile comprend un mélange d'huiles de basse viscosité et de haute viscosité selon un rapport compris entre 1/15 et 15/1 respectivement.

11. Une composition selon l'une quelconque des revendications 1 à 10, dans laquelle l'huile renferme une huile de faible tension superficielle choisie parmi les isotridécylisononanoate, PEG-4 diheptanoate, isostéarylnéopentanoate, tridécylnéopentanoate, cétyloctanoate, cétylpalmitate, cétylricinoléate, cétylstéarate, cétylmyristate, cocodicaprylate/caprate, décylisostéarate, isodécyloléate, isodécylnéopentanoate, isohexylnéopentanoate, octylpalmitate, dioctylmaléte, octanoate de tridécyle, myristate de myristile octyldodécanol, ou des mélanges d'octyldodécanol, lanolinique acétylé, acétate de cétyle, isododécanol et polyglycéryl-3-diisostéarate, et les mélanges de n'importe lesquels de ceux-ci.

12. Une composition selon l'une quelconque des revendications 1 à 11, qui comprend en outre un polypropylène amorphe ou atactique ayant une teneur atactique de 50 à 100 %, une cristallinité de 0,1 à 15 % et une masse moléculaire de 1000 à 10 000.

13. Une composition selon la revendication 12, dans laquelle le polypropylène atactique est choisi dans le groupe consistant en un polypropylène atactique ayant un point de ramollissement de 20°C et une masse moléculaire de 2000 ; un polypropylène atactique ayant un point de ramollissement de 135°C et une masse moléculaire de 5600, et un polypropylène atactique ayant un point de ramollissement de 150°C et une masse moléculaire de 4400.

14. Une composition selon l'une quelconque des revendications 1 à 13, comprenant :
(a) 35 à 60 % de solvant volatil qui comprend lui-même un rapport 10/1 à 1/10 de silicones cycliques et d'isoparaffines en C₈ à C₂₀;
(b) 3 à 10 % de lauryl-triméthylolpropanesiloxy silicate ou d'isostéaryltriméthylolpropane siloxy silicate ou de leur mélange;
(c) 10 à 30% de cire;
(d) 10 à 30 % de composant pulvérulent comprenant, en poids par rapport à la composition totale, de 10 à 20 % de poudre non pigmentée et 1 à 10 % de poudre pigmentée ; et
(e) 5 à 20 % de mélange d'huiles à faible et haute viscosités superficielles.

15. Un cosmétique comme un masque, fard à joues, un fard à paupières, un fond de teint ou un rouge à lèvres, ledit cosmétique comprenant une composition selon l'une quelconque des revendications 1 à 14, en association avec un réceptacle approprié pour celui-ci.

16. Une méthode de maquillage de la peau ou des lèvres, ladite méthode comprenant une administration topique d'une composition ou d'un cosmétique selon l'une quelconque des revendications 1 à 14.

17. Un procédé de préparation d'une composition cosmétique selon l'une quelconque des revendications 1 à 14, ledit procédé comprenant la mise en mélange physique intime de :
(a) 1 à 70 % de solvant volatil ayant une viscosité sensiblement dans la fourchette de 0,5 à 20 mPa.s à 25°;
(b) 0,1 à 15 % de cire d'ester de silicone qui est du lauryltriméthylopropane siloxy silicate ou de l'isostéaryltriméthylolpropane siloxy silicate ou un de leurs mélanges ;
(c) 10 à 45 % de cire ayant un point de fusion compris dans la fourchette de 35 à 120°C;
(d) 5 à 50 % de poudre ; et
(e) 1 à 30 % d'huile,
et, le cas échéant, en laissant le mélange ainsi obtenu prendre dans un
